# EUROPEAN PATENT SPECIFICATION

(11) **EP 3 212 072 B1**
(45) Date of publication and mention of the grant of the patent: **31.03.2021**
(21) Application number: 15865943.3
(22) Date of filing: 30.10.2015
(51) Int. Cl.: A61B 5/024, A61B 5/11, A61B 5/00

(54) **SLEEP MEASUREMENT COMPUTER SYSTEM**
COMPUTERSYSTEM ZUR SCHLAFMESSUNG
SYSTÈME INFORMATIQUE DE MESURE DU SOMMEIL

(30) Priority: 05.12.2014 US 201462087870 P
(43) Date of publication of application: 06.09.2017
(73) Proprietor: Apple Inc., Cupertino CA 95014 (US)
(72) Inventor: RIMMINEN, Henry, FI-02710 Espoo (FI); PAALASMAA, Joonas, FI-00180 Helsinki (FI); WARIS, Mikko, FI-00320 Helsinki (FI)
(74) Representative: Lang, Johannes
(86) International application number: PCT/FI2015/050751
(87) International publication number: WO 2016/087709

(56) References cited:
- US-A1- 2004 225 179
- US-A1- 2005 042 589
- US-A1- 2005 209 513
- US-A1- 2005 234 314
- US-A1- 2006 169 282
- US-A1- 2011 010 014
- US-A1- 2011 190 594
- US-A1- 2011 224 510
- US-A1- 2012 092 171
- US-A1- 2013 178 715
- US-A1- 2014 276 245
- PAALASMAA, JOONAS.: 'Monitoring Sleep with Force Sensor Measurement.' DEPARTMENT OF COMPUTER SCIENSE SERIES OF PUBLICATIONS, A REPORT A- 2014-2. pages 37-43 - 45-46, XP055446330 Retrieved from the Internet: <URL:http://urn.fi/URN:ISBN: 978-952-10-9723-2>
- KORTELAINEN J. M. ET AL.: 'Sleep Staging Based on Signals Acquired Through Bed Sensor.' IEEE TRANSACTIONS ON INFORMATION TECHNOLOGY IN BIOMEDICINE vol. 14, no. 3, 01 May 2010, pages 776 - 785, XP011327708

## Description

### TECHNICAL FIELD OF INVENTION

The present invention relates to a system for measuring sleep by detecting various sleep parameters. More particularly, the present invention is related to a sleep measurement computer system and method configured to deduce starting time of sleep.

### BACKGROUND

Sound sleep has always been considered vital for health of living beings. Abnormal sleep habits may lead to many health disorders. Some sleep disorders are very unsafe and may affect the physical and psychological functioning of human body. Further, even small quality improvements in sleep result in significant improvements in the overall quality of life and wellness over long time frames. Accordingly, various devices and tests are available for detecting, monitoring and/or recording sleep parameters.

The Applicant has devised a sleep measurement product that involves an App installed on the iPhone, which is in a wireless communication connection via Bluetooth with a sensor device that is placed underneath the mattress of the sleeping user. In this system, the user indicates by pressing an icon on the touch screen of the mobile phone that he starts to sleep, and thus the system begins measurement. Coincidentally, this icon is labelled "Start sleeping" in the Beddit App, which is publicly available in the Apple iOS App Store.

The current solution of the Applicant uses only piezoelectric sensors to measure the movement of the person on the bed.

The US patent application US2014/0269224 A1 discloses a system to determine the necessity of a snooze alarm by detecting whether the user is awake or asleep. The system determines at the current time when the user is asleep, but in doing so benefits from the recent data when the user was, in fact, asleep. This document is incorporated here as reference. The determination of the sleep state of a user is of course easier when history data from the whole night is at the disposal of the system as in the prior art.

Document US 2011/0 224 510 A1 describes systems and methods relating to a sleep diagnostic system including a mattress assembly, a plurality of sensors, data acquisition circuitry, an input device, a sleep processor, and a display device. The mattress assembly may have a sleeping surface, and the plurality of sensors may be disposed within the mattress assembly below the sleeping surface. The sensors are configured to measure at least one sleep condition and output data signals indicative of the sleep condition. The sleep processor receives signals from both the sensors and one or more other input devices, and determines a sleep characteristic based upon these received signals. The display device, which includes circuitry and a graphical user interface, receives the sleep characteristic from the sleep processor and displays it to the user.

Document US 2013/0 178 715 A1 describes a present-on-bed determination apparatus and sleep measurement apparatus.

Also, systems available in the prior art are designed only to be used by a single person and are incapable of determining the presence of more than one person starting sleep on a sleeping mattress.

In light of the above discussion yet unrealized technologies are needed that can be used to overcome one or more problems associated with the prior art.

### SUMMARY

The invention provides sleep measurement computer systems and sleep recording methods according to the independent claims. Further embodiments are provided in the dependent claims.

The invention under study is directed towards a system and a method for effectively measuring the sleep of at least one user. The present invention discloses a sleep measurement computer system comprising at least one sensor device that is arranged to be installed into the bed of a user. The sensor device comprises sensors configured to work, either alone or in combination. The at least one sensor device is further adapted to communicate with a mobile third party client application that is configured to measure sleep parameters of the user based on the communication with the at least one sensor device.

According to the invention, the mobile third party client application is configured to automatically deduce a starting time of sleep, based on data received from the at least one sensor device, without the user indicating the start of sleep manually. The data is received at a mobile subscriber terminal on which the mobile third party client application is installed and/or from any other peripheral device of the mobile subscriber terminal.

It is a further object of the invention to provide a sleep measurement computer system configured to automatically deduce the start time of sleep of a user without the need of any administrative support from the user for turning on sleep measurement manually.

According to the invention, the sleep measurement is arranged to be started on the occurrence of one or more start of sleep signaling events, either alone or in combination. The one or more sleep events signaling start of sleep include the detection of the entry of the user on the bed and may further include switching off the light by the user and/or entry of the mobile subscriber terminal to the proximity of one or more sensors.

According to the invention, the sleep measurement is arranged to be automatically ended when the at least one user exits the bed.

The sensors are the piezo thin film sensors that are further integrated with one or more sensor elements including a strain gauge, capacitive sensors, proximity sensors, thermocouples, inductive sensors and excited piezo thin film sensors

It is a further object of the invention to provide a sleep recording method for measuring sleep of at least one user. The method comprises initiating a communication channel between a sensor device and a mobile third party client application. Further, the sensor device communicates the sleep parameters to the mobile third party client application and the mobile third party client application automatically deduces the start of sleep time based on the sleep parameters received from the sensor device, without the need for the user to indicate start of sleep.

In other embodiments of the invention, the sensor device can communicate one or more sleeping parameter to the mobile third party client application including the user presence on the bed, time, sound levels of snoring, ballistocardio-graphic data, respiration rate, heart rate, skeletal muscle movement of the user while sleeping etc.

It is yet an object of the disclosure to provide a computer program product for recording the sleep of at least one user by a computing device, comprising at least one non-transitory computer-readable storage medium having computer-readable program code portions stored therein. The computer-readable program code portions including instructions are arranged to implement the aforementioned sleep recording method.

Further aspects of the disclosure relate to:

A sleep measurement computer system, comprising at least one sensor device comprising one or more sensors, the at least one sensor device arranged to be installed into the bed of at least one user is in accordance with the disclosure, wherein
- said at least one sensor device is adapted to communicate with a mobile third party client application, the mobile third party client being configured to measure sleep parameters of said at least one user based on the communication with the at least one sensor device; and
- said mobile third party client application being configured to automatically deduce a starting time of sleep, based on data received from the at least one sensor device, without said user indicating start of sleep, the data being received at a mobile subscriber terminal on which said mobile third party client application is installed and/or from any other peripheral device or incorporated device of said mobile subscriber terminal.

A sleep recording method to be implemented by a computing device, the method comprising the following steps is in accordance with the disclosure, comprising
- detecting occurrence of one or more sleep events of a user, either alone or in combination by one or more sensors;
- initiating a communication channel between the at least one sensor device and a mobile third party client application;
- communicating sleep parameters from the at least one sensor device to the mobile third party client application;
- automatically deducing start of sleep time based on the sleep parameters received from the at least one sensor device, without said user indicating start of sleep.

A computer program product for recording sleep of user by a computing device, the computer program product comprising at least one non-transitory computer-readable storage medium having computer-readable program code portions stored therein, said computer-readable program code portions comprising instructions in accordance with the diclosure, wherein
occurrence of one or more sleep events of a user is arranged to be detected, either alone or in combination by one or more sensors;
- a communication channel between the at least one sensor device and a mobile third party client application is arranged to be initiated;
sleep parameters from the at least one sensor device to the mobile third party client application are arranged to be communicated;
start of sleep time based on the sleep parameters received from the at least one sensor device is arranged to be automatically deduced, without said user indicating start of sleep.

According to an embodiment of the invention, the one or more sensors are arranged to form a matrix of sensors to be placed inside the fabric of bed. Preferably, the matrix of sensors is enabled to detect the presence of more than one user in the bed.

Some or all of the aforementioned advantages of the invention are accrued by the matrix of sensors arranged to detect the presence of more than one user in the bed for the measurement of sleep parameters of the at least one user.

In addition and with reference to the aforementioned advantage accruing embodiments, the best mode of the invention is believed to be represented by an embodiment in which one or more thin film piezo sensors are integrated with a plethora of different types of static sensors, namely the strain gauge sensor and the capacitive load cell sensor and the capacitive proximity sensor and the thermocouple and the inductive proximity sensor. There may be a plurality or just a single sensor of each type in the best mode. In the best mode the sensor device is arranged to communicate with the mobile third party client application, which application is arranged to determine the start of sleep automatically from sensor and/or other measurements without the user needing to manually specify when he starts to sleep. The combination of thin film piezo sensors (dynamic measurement technique) with a plurality of different static measurement techniques is especially preferable. This is because the combination of static measurement (is the user weighing on the bed) when combined with dynamic (is there a human moving on the bed) allows the computer system to accurately determine when in fact the user starts to sleep, without receiving any input from the user itself. The improved sensor technology in the best mode preferably allows the system to also automatically detect and distinguish between the time points of when the user goes to bed, i.e. turns of the lights and lies on the bed, and also when the user, whilst lying in bed, falls asleep. In the best mode, after the automatic detection of either the user in bed or the user falling asleep or both the recording of the sleep data begins automatically and the time spent lying in bed and the time spent asleep are preferably distinguished and displayed to the user.

### BRIEF DESCRIPTION OF THE DRAWINGS

In the following the invention will be described in greater detail with reference to exemplary embodiments in accordance with the accompanying drawings, in which:
FIG. 1 demonstrates an embodiment of the sleep measurement computer system 10 comprising a sensor device 120 that is adapted to communicate with a mobile third party client application 170 in accordance with the invention.
FIG. 2 demonstrates an embodiment 20 of the sleep measurement computer system with a strain gauge 210 integrated with a piezo thin film sensor in accordance with the invention.
FIG. 3 demonstrates an embodiment 30 of the sleep measurement computer system with a capacitive load cell sensor 310 integrated with a piezo thin film sensor in accordance with the invention.
FIG. 4 demonstrates an embodiment 40 of the sleep measurement computer system with a capacitive proximity sensor 410 integrated with the piezo thin film sensor in accordance with the invention.
FIG. 4B demonstrates an embodiment 41 of the sleep measurement computer system with a capacitive proximity sensor 410 integrated with the piezo thin film sensor with a different electrode design in accordance with the invention.
FIG. 5 demonstrates an embodiment 50 of the sleep measurement computer system with thermocouples 510 integrated with the piezo thin film sensor in accordance with the invention.
FIG. 6 demonstrates an embodiment 60 of the sleep measurement computer system with an inductive proximity sensor 610 integrated with the piezo thin film sensor in accordance with the invention.
FIG. 7 demonstrates an embodiment 70 of the sleep measurement computer system with a matrix of sensors 710 used to detect the presence of more than one user in the bed for the sleep measurement of at least one user in accordance with the invention.
FIG. 8 demonstrates a flowchart illustrating a sleep recording method in an embodiment 80 when a user is in the bed in accordance with the invention.
FIG. 9 demonstrates a flowchart illustrating a sleep recording method in another embodiment 90 when more than one user is in the bed in accordance with the invention.

The features of the invention illustrated above and below in the specification, are described with reference to the drawings summarized above. The reference numbers shown in the drawings may be used at one or more places to indicate the functional relation between the referenced elements. Some of the embodiments are described in the dependent claims.

### DETAILED DESCRIPTION OF EMBODIMENTS

It should be noted that the terms "a" or "an," as used herein, may be defined as one or more than one. The term "another," as used herein, is defined as at least a second or more. The terms "including" and/or "having" as used herein, are defined as comprising i.e. open transition.

In addition, features, functions and mechanisms described herein are not limited to any particular sequence, and the blocks or states relating thereto can be performed in other sequences that are appropriate. For example, described blocks or states may be performed in an order other than that specifically disclosed, or multiple blocks or steps may be combined into a single block or step.

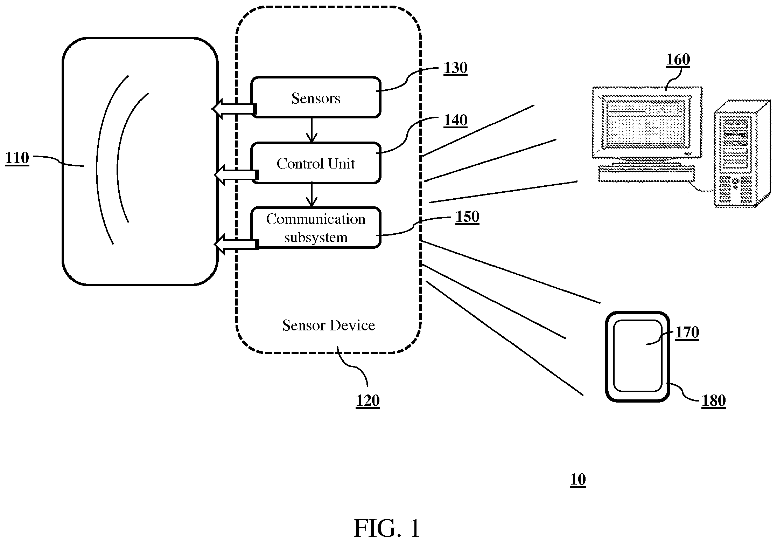

Referring to FIG. 1, in accordance with an embodiment of the invention, there is shown a sleep measurement computer system 10. The sleep measurement computer system 10 includes at least one sensor device 120 installed into a bed 110 to detect at least one of the sleep parameter of at least one user. The sensor device 120 further includes one or more sensors 130, a control unit 140 and a communication subsystem 150. The one or more sensors 130 of the sensor device 120 are preferably piezo thin film sensors that are configured to work alone or in combination with similar type sensors or various other sensors of different types. The sensor device 120 is further arranged to communicate with a mobile third party client application 170 being installed on a computing device. In an embodiment of the invention, the computing device is a mobile subscriber terminal 180 that is arranged to communicate with the sensor device 120 and measure sleep parameters of the at least one user.

In another embodiment of the invention, the computing device is computer system 160. In other embodiments of the invention, the computing device may be any device including but not limited to mobile phone, tablet, personal computer and any other device now known or later developed.

In yet another embodiment of the invention, the communication subsystem 150 is configured to enable communication between the at least one sensor device 120 and the mobile third party client application 170 stored on the mobile subscriber terminal 180. The communication starts when the communication subsystem initiates a communication channel between the sensor device 120 and the mobile subscriber terminal 180. The communication subsystem 150 is configured to utilize the wired or wireless networks that may include, but not limited to CDMA, GSM, UMTS, HSPA, EV-DO, EV-DO rev. A, 3 GPP LTE, WiMAX, Wi-Fi, Bluetooth, Low Energy Bluetooth, Internet, telephony, or other communication formats including combinations thereof.

In a further embodiment of the invention, the mobile third party client application 170 is arranged to automatically deduce a starting time of sleep, based on data received from the at least one sensor device 120, to a mobile subscriber terminal 180 in which the mobile third party client application 170 is installed and/or from any other peripheral device of said mobile subscriber terminal without said user indicating start of sleep actively.

According to an embodiment of invention, the sleep parameters are the parameters including but not limited to the user presence on the bed, time, sound level of snoring, ballistocardio-graphic data, respiration rate, heart rate, light and/or intensity in the proximity of the user, sensor and/or mobile subscriber terminal and skeletal muscle movement of the at least one sleeping user etc. A photodetector may be placed on/in the mobile subscriber terminal or sensor device to detect light intensity levels, e.g. the event when lights are turned off from the room in selected embodiments. In some embodiments a photodetector already present in the mobile subscriber terminal, for example a detector associated with the brightness control of the display, may be used. Similarly a sound detector can be realized either in the mobile subscriber terminal or in the sensor device to detect sound intensity levels. It is usually silent for a while when sleeping starts, after which the snoring may start. In some embodiments the existing microphone in the mobile subscriber terminal is used to detect sound levels. For example in some embodiments the microphone input signal is read by the 3^{rd} party application, via an API (Application Programmer Interface) or otherwise to determine sound levels.

In a further embodiment of invention, the sensor device measures signals obtained from one or more sensors at certain times and sends data to a communication hub at certain times (e.g. the user's mobile device). The communication hub may be a personal computing system or may be a centralized computer system.

It should be noted that any features, phases or parts of embodiment 10 can be freely permuted and combined with any of the embodiments 20, 30, 40, 41, 50, 60, 70, 80, and/or 90 in a combination of two or more embodiments in accordance with the invention.

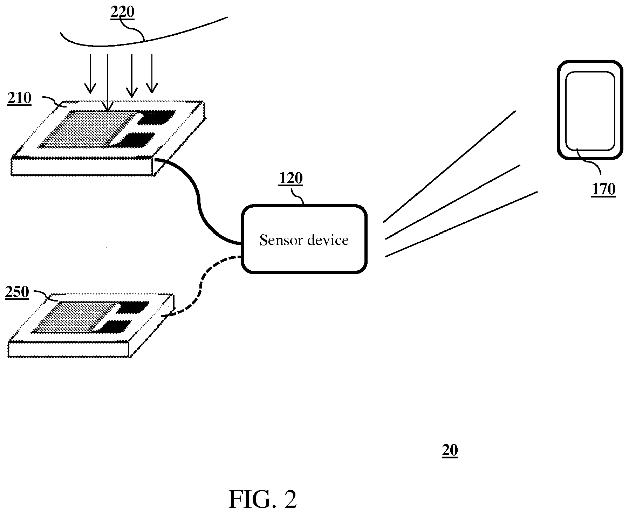

In the following Figures 2-9 the strain gauge and all of the other following measurement methods have advantage over the piezoelectric measurement, since they have capability to detect static presence of the person, unlike the piezoelectric measurement, which can only detect dynamic phenomena. Static presence indication is very preferably used to deduce starting time of sleep. These sensors, excluding the piezoelectric measurement, are referred to as static measurement sensors in this application.

The piezoelectric measurement has superior sensitivity for dynamic phenomena, so it is the primary method for vital sign detection and sleep measurement. Even though having no static presence detection capability, the variability of the piezoelectric output is also used as a presence indicator in some embodiments, since the human body is never completely still.

Preferable embodiments of the invention are currently envisioned to be found in the combinations of the piezoelectric measurement with any of the other measurement technologies discussed with Figures 2-9 and embodiments 20-90. However, other embodiments may be good and practical also in accordance with the invention.

Referring to FIG. 2, in an embodiment 20 of the invention, a strain gauge 210 is shown to be integrated into the film of one or more sensors of the sensor device 120. The strain gauge 210 is used to detect the state of physical motion of the user 220 on the bed, by detecting the static deflection of the sensor caused by the presence of the person. The strain gauge 210 integrated into the piezo sensors may measure the vibration signals occurring due to the respiration and/or other body movements of the user 220. As depicted in FIG. 2, the sensor device 120 may include other strain gauge 250 being integrated with the one or more sensors to detect the sleep parameters.

In some embodiments the strain gauge sensor is arranged on the elastic fabric of user mattress in such a way that movements of the user's chest due to heart beat and/or respiration lead to a fluctuating strain gauge signal. The direct signal originating from the strain gauge in order to monitor heart beat and respiration may be communicated to the mobile third party client application 170.

According to a further embodiment of the invention, the strain gauge 210 is provided in the piezo thin film sensor. Generally, strain gauge 210 consists of a flexible film that can be incorporated with the thin film of piezo thin film sensors. However, according to yet another embodiment of the invention, two or more strain gauges 210, 250 are provided in the piezo thin film sensors. This provides for a higher sensitivity to measure the sleep parameters. This has the advantage that the user's static presence becomes available. For example, if high fluctuations in the strain gauge signals arc dctcctcd, a serious health condition may be assumed by the mobile third party client application 170.

It should be noted that any features, phases or parts of embodiment 20 can be freely permuted and combined with any of the embodiments 10, 30, 40, 41, 50, 60, 70, 80, and/or 90 in a combination of two or more embodiments in accordance with the invention.

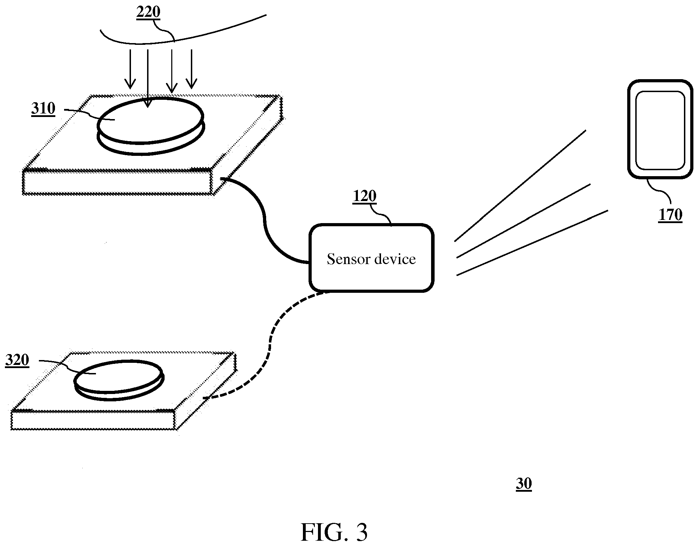

In another embodiment 30 of the invention as depicted in FIG. 3, one or more sensors are integrated with one or more capacitive load cells 310, 320 that include a capacitive foam material placed between two metal electrode films. The capacitive load cell 310, 320 integrated with one or more sensors are further configured to be integrated into a fabric of the bed in some embodiments. In one embodiment of the invention, the capacitive load cell 310, 320 includes a capacitive foam material of thickness 1.0 mm that is placed between two metal electrode films.

In an exemplary embodiment, two capacitive load cells 310, 320 may be mounted at or near the top and bottom middles of the user' s bed to detect the presence of user by detecting an increment in the weight on the bed. Various algorithms may utilize the various combinations of the capacitive load cell 310, 320 to detect the weight of the user 220 on the bed, exit of the user from the bed and the presence of more than one user 220 on the bed. The output signals of the capacitive load cells, which may typically be arranged as voltage or current level outputs, may be digitized for processing by a control unit of the sensor device 120 and further communicated to the mobile third party client application 170 in some embodiments.

It should be noted that any features, phases or parts of embodiment 30 can be freely permuted and combined with any of the embodiments 10, 20, 40, 41, 50, 60, 70, 80, and/or 90 in a combination of two or more embodiments in accordance with the invention.

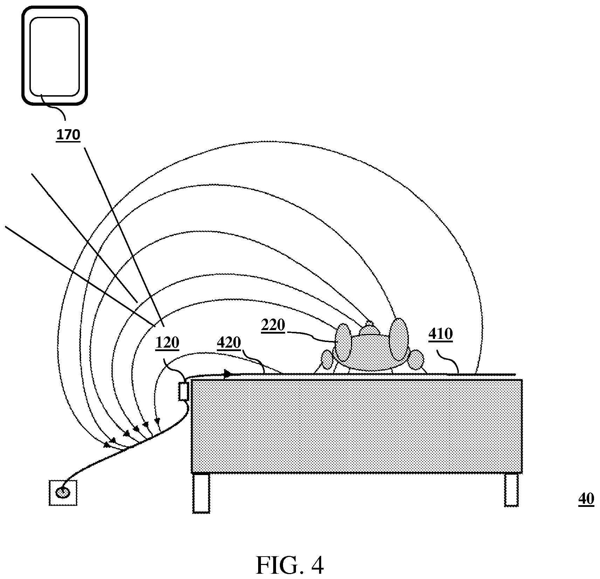

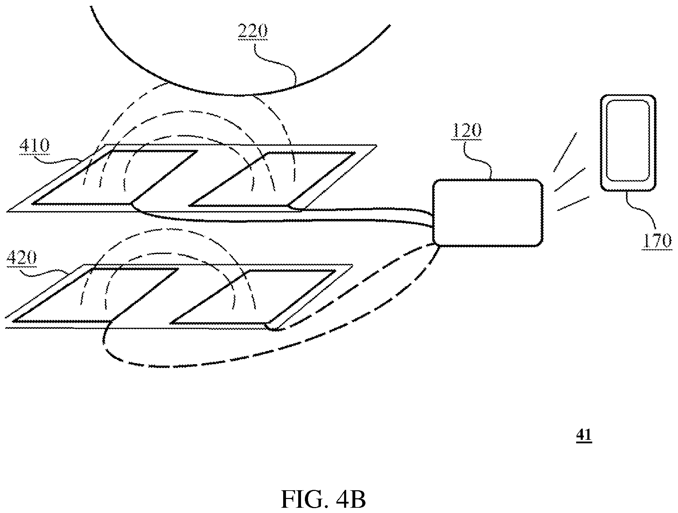

In another embodiment 40 of the invention as depicted in the schematic diagram FIG. 4, which is not to scale, a capacitive proximity sensor film 410, 420 is integrated into the one or more sensors. In practice, the film is very thin, typically millimeters in thickness, as shown for proximity sensor films 420, 410. The capacitive proximity sensor film 410, 420 is used to detect the presence of user and/or mobile terminal in the proximity of one or more sensors. For example, the presence of the user 220 alters the electric flux field lines of at least one capacitive proximity sensor films 410, 420 which is detected. Typically the electric flux density increases when the user 220 lies on top of the capacitive proximity sensor film 410, 420. The technology is analogous to touch screen sensors. One capacitive proximity sensor film 410 or a plurality of capacitive proximity sensor films 410, 420 can be used in accordance with the invention.

In the embodiment of the invention, the sensor device 120 includes two capacitive proximity sensors 410, 420 housed inside the mattress of bed; the two capacitive proximity sensors 410, 420 are further interconnected with a control unit of the sensor device 120. The control unit senses variations in the capacitance or impedance value within the capacitive proximity sensor induced through the dielectric shifts within the one or more sensors due to the presence and absence of user 220 in the proximity of the one or more sensors.

In one preferred embodiment of the invention, the one or more sensors are piezo thin film sensors. Various algorithms can be employed to continually analyze and optimize signals received from and generated by the sensor device 120. In this way, the sleep measurement computer system defines capacitive value parameters by which it interprets whether a user 220 is present in the proximity of sensors or not. In such manner, the mobile third party client application 170 determines the presence or absence of a user on the bed. Further, the algorithms can be employed to measure the sleep of more than one user by detecting the presence of more than one user. For example, if the capacitive value parameters are higher than the parameters examined for the one user, the mobile third party client application 170 may determine the presence of more than one user in the proximity of one or more sensors.

It is in accordance with the invention to incorporate the capacitive proximity sensor into the piezo thin film sensor, thereby producing a sensor film with dynamic and static detection capabilities in the same sensor film. Both of these sensor types, individually, separately or in combination may also be integrated with the bed, mattress or any fabric in accordance with the invention. In one preferable embodiment where the capacitive proximity sensor and the piezo thin film sensor are integrated, the electrodes of the piezoelectric sensor are used in the capacitive measurement. This feature allows for a simple and integrated design for the dual mode (static and dynamic) sensor device and sensor film. The static presence is simply measured by producing an electric field with the electrodes at certain intervals and measuring whether the impedance, (i.e. the dielectric properties) change with the introduction of a person on the bed. It is also possible that different dedicated electrodes are provided for the static capacitance measurement sensor in accordance with the invention.

It should be noted that any features, phases or parts of embodiment 40 can be freely permuted and combined with any of the embodiments 10, 20, 30, 41, 50, 60, 70, 80, and/or 90 in a combination of two or more embodiments in accordance with the invention.

Figure 4B shows an alternative embodiment of the sensor setup of Figure 4. In this embodiment both the transmitting and the receiving electrodes are integrated with the piezo thin film sensor, which sensor is installed in bed.

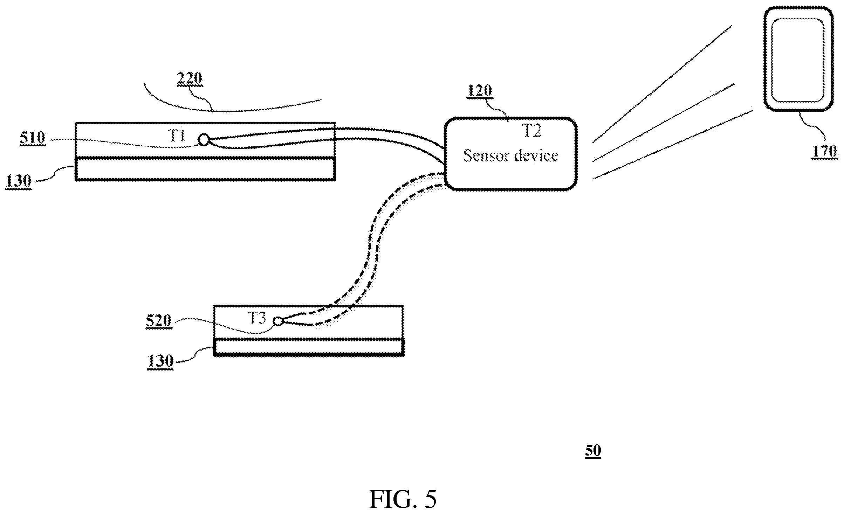

In another embodiment 50 of the invention as depicted in FIG. 5, one or more thermocouples 510, 520 are integrated into the one or more sensors. In the embodiment of the invention, the one or more sensors of sensor device 120 are enabled to measure temperature differences between the one or more thermocouples 510, 520 and the sensor device 120. The temperature difference between the terminals of thermocouples indicates presence of one user 220, or more than one user.

In some embodiments, the sensor device 120 is outside the bed, so T2 is close to ambient temperature of the room. This arrangement rejects the unwanted disturbance caused by changes in the ambient temperature. The absolute temperature at point T1 is calculated by adding the difference T1-T2 to the absolute temperature at the sensor device 120, which is measured with a separate absolute temperature meter inside the sensor device 120.

One or more thermocouples 510, 520 temperatures are preferably compared the ambient temperature T2 in accordance with the invention. However, sometimes the absolute temperatures at T1 and T3 can be used to determine whether one or more people are sleeping in the bed.

A large temperature change may indicate that only one half of a double bed is occupied, whereas a small difference in two measurements both close to human body temperature of 37 Celsius degrees may indicate two people sleeping in the double bed. Typically, temperatures close to 37 Celsius degrees indicate presence of a person directly on top of one thermocouple. With two thermocouples on both sides of a double bed, the information can be used to determine if no persons are present, two persons are present or just one person is present on left or on right side of the bed.

According to an exemplary embodiment, there is provided herein the thermocouple films incorporated in to the one or more thin film piezo sensors that can detect temperature at multiple points within the bed of the user. Various algorithms can be employed to check a temperature variation for the purpose of detecting the presence of at least one user and further for recording the sleep of at least one user. In some instances, for example, when the user changes position or leaves the bed, the changes in temperature are also measured by the mobile third party client application 170. Thus, when a temperature change is detected the instant invention will determine whether the change is due to movement, i.e. the presence or absence of the user 220 from the bed. Also in some embodiments, when a temperature exceeds a predefined or dynamic threshold, the instant invention may determine whether it is caused by presence of the user 220 in the bed.

Further, some embodiments of the invention can be employed to record the temperature of the user using thermocouples for monitoring the health of the user in the sleeping stage. This might be interesting especially in situations where the patient has a fever.

It should be noted that any features, phases or parts of embodiment 50 can be freely permuted and combined with any of the embodiments 10, 20, 30, 40, 41, 60, 70, 80, and/or 90 in a combination of two or more embodiments in accordance with the invention.

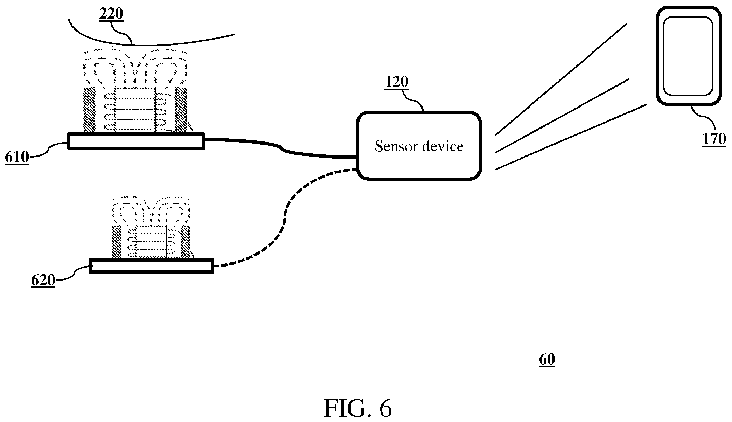

In another embodiment 60 of the invention as depicted in FIG. 6, an inductive proximity sensor 610 is integrated into the one or more sensors of the sensor device 120 to detect presence of the at least one user 220 by creating an AC magnetic field around the one or more beds, users, sensors and/or sensor device 120. The presence of the user 220 generates Eddy current losses. The inductive proximity sensors 610, 620 are enabled to detect changes in the electromagnetic field introduced by the user 220 entering or exiting the proximity of one or more sensors.

In a still further embodiment of the invention, the piezo film in the sensor is excited with an external power source, causing the piezo film to vibrate. Typically the piezo film is driven with alternating voltage or current at a frequency of 30-60 kHz.

The piezo film is integrated into the one or more sensors to induce movement in the thin film of the piezo thin film sensors, which movement is caused by the alternating voltage or current at a frequency of 30-60 kHz. In the embodiment, the excited piezo thin film sensor element is placed in the mattress of the bed. The motional resistance of the moving sensor changes when it is moving freely and when the body of the person is on top of it. The weight of the person causes damping in the motion.

The change of the motional resistance is detected by measuring the impedance of the piezo film while exciting it with AC voltage or current. These film sensors are similar to the piezo film displayed in Figure 2 in appearance. The same film can be used for sleep measurement and excited with alternating voltage in a sequence.

In a still further embodiment of the invention, an excited piezo film with alternating voltage 30-60 kHz is integrated into the one or more sensors to induce movement in the thin film of the piezo thin film sensors. In the embodiment, the excited piezo thin film sensor element is placed in the mattress of the bed. The one or more sensors are employed to detect the small mechanical movements. The user 220 may have some low frequency movements generated due to the blood pressure variations and muscle motion etc. In the embodiment of the invention, the mobile third party client application 170 can determine blood pressure and other low frequency variations in the user 220 body. In one embodiment, two excited piezo film sensors can be configured for a non-invasive velocity type blood pressure monitoring.

It should be noted that the magnets in Figure 6 are not to scale, and in most embodiments the coil would be planar, resulting in a planar sensor, which is the preferred shape of the sensor.

It should be noted that any features, phases or parts of embodiment 60 can be freely permuted and combined with any of the embodiments 10, 20, 30, 40, 41, 50, 70, 80, and/or 90 in a combination of two or more embodiments in accordance with the invention.

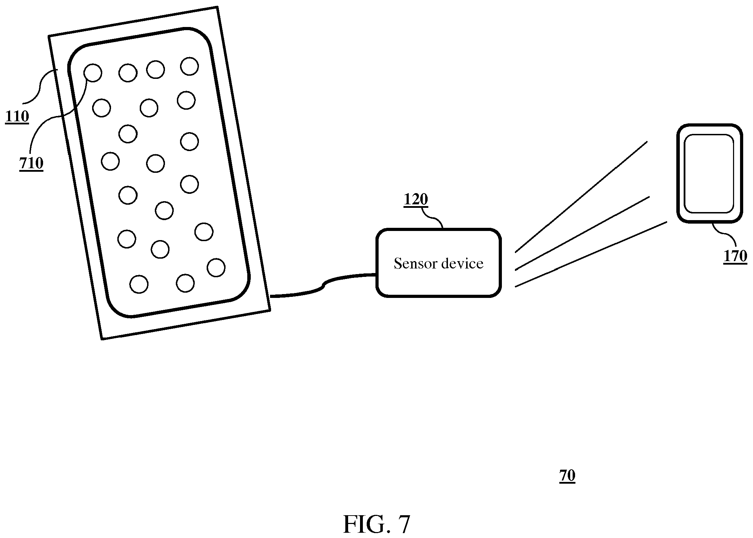

In another embodiment 70 of the invention as depicted in FIG. 7, the at least one sensor device 120 includes a sensor matrix 710 that is configured to be placed inside the fabric of the bed 110 to detect the presence of one or more user in the bed 110. The sensor matrix 710 may be designed with the one or more sensors as disclosed in previous embodiments for detecting various sleep parameters. The sensor matrix 710 may be stretched to fit the entire bed so that it may effectively detect the presence of one or more users anywhere in the bed.

In an exemplary embodiment of the invention, the sensor matrix 710 is fitted under the mattress of the bed. In some other embodiments of the invention the sensor matrix 710 is a matrix of thin film sensors that can be printed on the user mattress. The sensor device 120 is enabled to communicate the sleep parameters collected from the sensor matrix to the mobile third party client application 170.

It should be noted that any features, phases or parts of embodiment 70 can be freely permuted and combined with any of the embodiments 10, 20, 30, 40, 41, 50, 60, 80, and/or 90 in a combination of two or more embodiments in accordance with the invention.

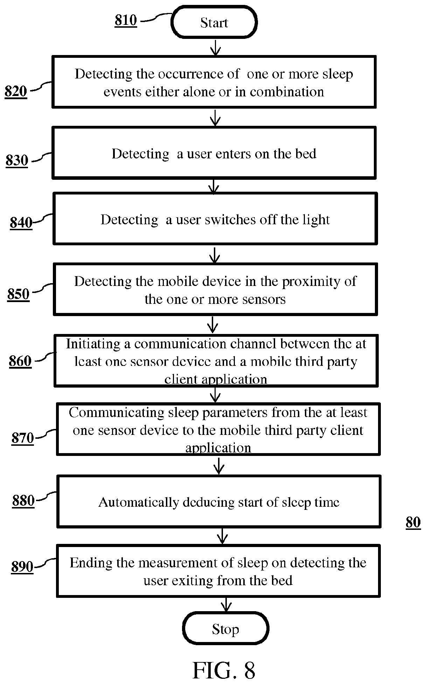

As depicted in FIG. 8, an embodiment 80 of the invention discloses a sleep recording method to be implemented by a computing device.

The method initiates at step 810. The method may be perpetually on in the mobile subscriber terminal, for example so that step 810, and possibly any number or all of the following steps takes place without the 3^{rd} party application being open in the operating system of the mobile subscriber terminal.

At step 820, the one or more sensors according to the embodiments disclosed above detect the occurrence of one or more sleep events. The sleep events may include one or more events including detection of a user's entering in the bed; detection of user switches on the light; and the detection of a mobile device in the proximity of the one or more sensors.

In the next step 830, the at least one sensor device 120 detects the entering of a user in the bed. This is typically detected by pressure sensors as discussed before with embodiments 10-70 in Figures 1-7.

Thereafter, at step 840, the at least one sensor device 120 detects the switching off the light by the user. This is typically done by reading any light intensity level measurements from a photodetector, which may either be arranged into the sensor device or the mobile subscriber terminal. In some embodiments the camera API allows the third party application to read light intensity data detected by the camera photodetector, and this data is used to determine whether lights have been turned off or not in the surroundings of the user, sensor device and/or bed.

Similarly, if step 840 is replaced or complemented by sound level detection measurements, the API of the microphone of the mobile phone can be used in some embodiments to read the sound levels in the surroundings of the mobile subscriber terminal into the 3^{rd} party application.

In step 850, the presence of a mobile device is detected in the proximity of one or more sensors. In some embodiments iBeacon technology, which works via Low Energy Bluetooth, is used to determine the proximity and/or distance between the sensor device and the mobile subscriber terminal.

Thereafter, at step 860, the computing device initiates a communication channel between the at least one sensor device 120 and the mobile third party client application 170. This is typically established also wirelessly, preferably via Low Energy Bluetooth.

At step 870, the at least one sensor device 120 communicates the sleep parameters to the mobile third party client application 170. The data is preferably carried over the wireless connection, which is typically a radio connection, and which radio connection is preferably Low Energy Bluetooth as discussed before, or can be any other wireless and/or radio connection, such as WIFI or cellular also.

In a further step 880, the mobile third party client application 170 automatically deduces the start of sleep time based on the sleep parameters received from the at least one sensor device 120, without user indication of start of sleep. This has great operational advantages over long time frames. The most common reason why users do not get their sleep data is due to the fact that they forget to record it. Also, even if the user remembers to turn sleep recording on, it is one administrative task that burdens the user further every day.

In step 890 the user exits the bed, the exit is detected and subsequently sleep measurement is stopped.

It should be noted that any features, phases or parts of embodiment 80 can be freely permuted and combined with any of the embodiments 10, 20, 30, 40, 41, 50, 60, 70, and/or 90 in a combination of two or more embodiments in accordance with the invention.

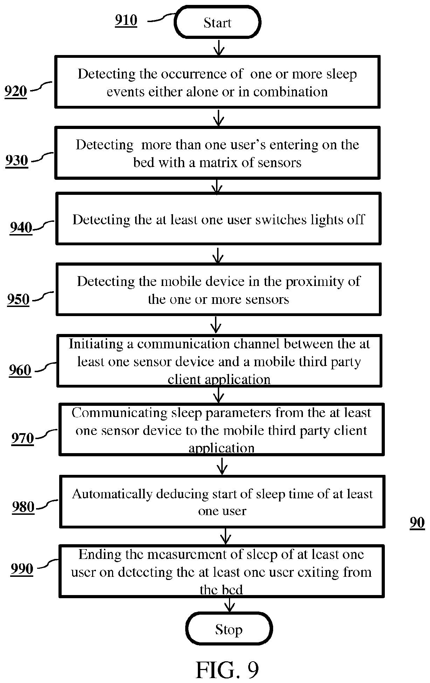

In a further embodiment 90 of invention, as depicted in FIG. 9, the invention provides a method for recording sleep of more than one user.

The method 900 initiates at a step 910. The method may be perpetually on in the mobile subscriber terminal, for example so that step 910, and possibly any number or all of the following steps take place without the 3^{rd} party application being open in the operating system of the mobile subscriber terminal.

In step 920, the one or more sensors according to the embodiments disclosed above detect the occurrence of one or more sleep events. The sleep events may include one or more events including detection of more than one user' s entering in the bed with a matrix of sensors; detection of at least one user switches on the light; and the detection of a mobile device in the proximity of one or more sensors.

In next step 930, the entering of the at least one user is detected on the bed. This is typically detected by pressure sensors as discussed before with embodiments 10-70 in Figures 1-7.

In a further step 940, the switching off the light by the at least one user is being detected. This is typically done by reading any light intensity level measurements from a photodetector, which may either be arranged into the sensor device or the mobile subscriber terminal. In some embodiments the camera API allows the third party application to read light intensity data detected by the camera photodetector, and this data is used to determine whether lights have been turned off or not in the surroundings of the user, sensor device and/or bed.

Similarly, if step 940 is replaced or complemented by sound level detection measurements, the API of the microphone of the mobile phone can be used in some embodiments to read the sound levels in the surroundings of the mobile subscriber terminal into the 3^{rd} party application.

In step 950, the presence of a mobile device is detected in the proximity of one or more sensors. In some embodiments iBeacon technology, which works via Low Energy Bluetooth, is used to determine the proximity and/or distance between the sensor device and the mobile subscriber terminal.

In next step 960, the computing device, which is typically a mobile subscriber terminal, initiates a communication channel between the at least one sensor device 120 and a mobile third party client application 170. This is typically established also wirelessly, preferably via Low Energy Bluetooth between the computing device and the sensor device.

In step 970, the at least one sensor device 120 communicates the sleep parameters to the mobile third party client application 170. The data is preferably carried over the wireless connection, which is typically a radio connection, and which radio connection is preferably Low Energy Bluetooth as discussed before, or can be any other wireless and/or radio connection, such as WIFI or cellular also.

In further step 980, the mobile third party client application 170 automatically deduces the start of sleep time of at least one user based on the sleep parameters received from the at least one sensor device 120, without the at least one user indicating the start of sleep. This has great operational advantages over long time frames. The most common reason why users do not get their sleep data is due to the fact that they forget to record it. Also, even if the user remembers to turn sleep recording on, it is one administrative task that burdens the user further every day.

In step 990 one user exits the bed, the exit is detected and subsequently sleep measurement is stopped for the exiting user. Sleep measurement can be continued for the user who remains in the bed asleep in some embodiments.

An embodiment of the invention provides a computer program product for recording the sleep of at least one user by the computing device. The computer device includes at least one non-transitory computer-readable storage medium having computer-readable program code portions stored therein. The said computer-readable program code portions comprise instructions to implement the aforementioned sleep recording method. The exemplary methods described above are typically stored on a computer-readable storage medium, which may be any device that can store code for use by a computer system, mobile computer system and/or other computer systems. The computer-readable storage medium includes, but is not limited to volatile memory, non-volatile memory, magnetic and optical storage devices such as disk drives, magnetic tape, CDs compact discs, DVDs digital versatile discs or digital video discs, or other media capable of storing code now known or later developed.

Furthermore, methods described herein can be embossed on hardware modules or apparatus. These modules or device may include, but are not limited to, an application-specific integrated circuit ASIC chip, a field-programmable gate array FPGA, a dedicated or shared processor that executes a particular software module or a piece of code at a particular time, and/or other programmablc-logic devices known or later developed. When the hardware modules or apparatus are activated, they perform the methods and processes included within them.

It should be noted that the invention can be used to automatically detect, without user input: a) the time when the user goes to bed and/or b) when the user falls asleep in the bed. The automatic detection of a) or b) or both may be used to begin automatic recording of sensor data into a data storage device for sleep measurement. The data storage device may be within the sensor, in the mobile subscriber terminal and/or over a computer network in accordance with the invention.

It should be noted that in some embodiments of the invention, the location of the mobile subscriber terminal may be used as a proxy for the user location, and that location is used to determine the presence of the user, for example at the location on top of the bed or on top of the sensor or otherwise close by. In some embodiments of the invention, the location of the mobile subscriber terminal is obtained from one or more iBeacons, one or more WIFI base station, cellular network base stations or a satellite system such as GPS. The same or similar locating solutions may be also used to locate the sensor device in those embodiments wherein the sensor is compatible communicating with iBeacon base stations, WIFI base stations, cellular base stations and/or satellite based locating systems such as GPS. In some embodiments of the invention the aforementioned location data could be solely used to initiate the automatic sleep data measurement without user input.

It should further be noted that any of the embodiments of the invention can feature an air moisture and/or air humidity detector as a sensor in/on the bed. Typically the presence of a sleeping user on the bed increases air humidity due to e.g. sweating, respiration etc. The change in air humidity could be used in some embodiments to detect the start of sleeping by the user, and thus the change in air humidity could be configured to start the automatic recording of sleep measurement data without user input. It is known that the sleep cycle of a person influences body temperature, which in turn influences the humidity in direct proximity of the user. Therefore, the humidity measurement could be used in detecting automatically when the person falls asleep when in bed. Further, the humidity measurement can be used in the recording of sleep data during the night to define the sleep cycles of the user more accurately.

It should be noted that any features, phases or parts of embodiment 90 can be freely permuted and combined with any of the embodiments 10, 20, 30, 40, 41, 50, 60, 70 and/or 80 in a combination of two or more embodiments in accordance with the invention. Further, it should be noted that any features, phases or parts of any embodiment 10, 20, 30, 40, 41, 50, 60, 70, 80, and/or 90 can be freely permuted and combined with any of the embodiments 10, 20, 30, 40, 41, 50, 60, 70, 80, and/or 90 in a combination of two or more embodiments in accordance with the invention.

Some or all of the aforementioned advantages of the invention are accrued by the matrix of sensors arranged to detect the presence of more than one user on the bed for the measurement of sleep. The matrix of sensors can be placed in an elastic fabric, so that the fabric could be stretched to span the width of the whole double bed, or only a part of it. The advantage of this would be that the sensor can be installed properly to beds with many widths. The best mode of the invention is believed to be represented by an embodiment in which one or more thin film piezo sensors are integrated with a plethora of different types of static sensors, namely the strain gauge and the capacitive load cell sensor and the capacitive proximity sensor and the thermocouple and the inductive proximity sensor. There may be a plurality or just a single sensor of each type in the best mode.

Further, another preferred mode of the invention is where the sleep parameters are detected by the one or more sensors being integrated with the one or more sensor elements including but not limited to the strain gauge, capacitive sensors, proximity sensors, thermocouples, inductive sensors, excited piezo film and their combination thereof. The mobile third party client application 170 is enabled to record the sleep of at least one user using the one or more sleep parameters detected by the sensor elements. The combination of above mentioned sensor elements used in determination of the one or more sleep parameters enables the sleep measurement computer system to measure sleep of at least one user more precisely. Further, various algorithms are used to analyze the sleep of at least one user and to determine the accurate sleep start time of the user using the combination of various sleep parameters. The mobile third party client application 170 is configured to analyze the sleep and to deduce the sleep start time of at least one user without the need of user indication for start of sleep.

In some other embodiments of the invention, the mobile third party client application 170 may be configured to determine the sleep time, sleep cycle and health related data of user by measuring one or more sleep parameters of at least one user without any administrative support for starting the sleep measurement. The mobile third party client application 170 may further be configured to generate sleep reports of one or more users. The mobile third party client application 170 may also communicate the user sleep reports to one or more centralized hubs such as hospitals and clinics. The mobile third party client application 170 can also be configured to share user sleep data with one or more mobile applications for further analysis.

It should be noted that in some embodiments of the invention the mobile subscriber terminal 180 may include the subscriber terminal along with its accessory, e.g. an iPhone and Apple Watch from Apple Computers. That is, the mobile subscriber terminal 180 is to be understood to include a tablet, a mobile phone and/or a wrist computer, such as Apple Watch, each separately or in combination. The mobile subscriber terminal 180 of the invention may also have mobile third party client application software 170 distributed on multiple devices, like e.g. the iPhone and the Apple Watch have software distributed among one another. In other words, the mobile third party client application 170 may be installed and operable in a wrist computer (for example Apple watch) alone, or in a mobile phone (for example iPhone) alone, or the mobile third party client application 170 may have parts of itself installed and/or operable in both the wrist computer 180 and the mobile phone 180 in accordance with the invention.

In some embodiments of the invention the events indicating an intent to sleep and the start of actual sleep are distinguished. For example, in one embodiment the bed has a piezo and/or capacitive sensor 120, 130, which detects automatically the entry of the person into the bed. Based on this, the sensor 120, 130 notifies a network element, such as an iBeacon, that the person is in bed, at which point the mobile subscriber terminal 180 begins recording sleep signals after having received the signal from the network element, such as the iBeacon, to do so. Of course the sensor 120, 130 can also notify the mobile subscriber terminal 180 also directly, without a network element in between, in many embodiments of the invention. Only after receiving recorded data, the starting time point of sleep is determined, as the person does not sleep immediately when the user enters the bed. That is, there arc sleep events that indicate intention to sleep, start of sleep or both. It should be also noted that while the piezoelectric sensors or the piezo thin film sensor are dynamic, they can be used to trigger the start of measurement based on the signal pulse created by the person entering the bed. In other words, the piezo based sensor can also be used to detect intentions of sleeping, in addition to sleeping itself.

In some special embodiments, the sensor and the iBeacon are incorporated together into the sensor device 120, 130, which device is placed in the bed. Then, typically the sensor detects the start of sleep and produces a signal to the incorporated iBeacon. The iBeacon then sends a signal, typically via Bluetooth, to the mobile subscriber terminal 180, such as an iPhone, to begin recording sleep. Subsequently, the mobile subscriber terminal 180 opens a communication connection to the sensor and/or the iBeacon 120, 130 and begins recording sleep data produced by the sensor via the established communication connection. Preferably the whole process of this paragraph takes place automatically in accordance with the invention.

Additionally, the start of sleep may be determined based on any of the following supporting signals:
- mobile subscriber terminal 180 accelerometer detects that the terminal stops moving and/or mobile subscriber terminal accelerometer detects that the terminal is not in the user's pocket or hand,
mobile subscriber terminal 180 display is switched off,
user stops interacting with the mobile subscriber terminal 180 user interface,
mobile subscriber terminal 180 microphone detects silence,
user connects mobile subscriber terminal 180 or related accessory device such as Apple Watch to the battery charger,
user takes mobile subscriber terminal 180 or related accessory device, such as watch or Apple Watch off from wrist or hand, and/or
- time of day indicates that user's normal bedtime is approaching.

The aforementioned events may be used to detect the start of sleep in many embodiments of the invention. Many times detecting a combination of the said supporting signals is needed to determine that the user has started sleeping. The supporting signals listed here may be used also in combination with any other earlier mentioned methods of detecting sleep in accordance with the invention.

Further, the end of sleep may be determined based on any of the following supporting signals:
- user switches on lights, or uses connected appliances or services, which can be detected e.g. with a luminosity sensor, Apple HomeKit connected device or similar device,
- user disconnects mobile subscriber terminal 180 from charger,
- user switches mobile subscriber terminal 180 display on,
- mobile subscriber terminal 180 accelerometer detects movement,
- mobile subscriber terminal user starts using said mobile subscriber terminal 180,
- user starts using a connected accessory to the mobile subscriber terminal, such as a wrist computer 180, e.g. Apple Watch,
- user starts using some other device connected with the mobile third party client application (170) (such as a tablet or watch),
- the usual wakeup time of the user is approaching, and/or
- user is active and/or starts being active in at least one connected third party service, such as Facebook.

The aforementioned events may be used to detect the end of sleep in many embodiments of the invention. Many times detecting a combination of the said supporting signals is needed to determine that the user has woken up. The supporting signals listed here may be used also in combination with any other earlier mentioned methods of detecting the user waking up in accordance with the invention.

In some embodiments of the invention a more accurate start measurement detection mode is activated. For example, when some indicator, such as a strong push by the user on the mobile subscriber terminal 180, is applied, the mobile third party client application 170 can (without starting actual detailed sleep measurement) use sensor inputs 120, 130 to make a more accurate decision on whether to start measurement or continue sleep measurement.

Further, it is possible in all embodiments of the invention that the sleep measurement automatically ends when the mobile subscriber terminal 180 moves away from the proximity sensor. Further, it is also possible that in all embodiments the measurement starts by detecting the user entering the bed, and subsequently after this the communication channel between the sensor device and the mobile third party client application 170 is opened.

Even further, the sensor devices of the invention typically have a thickness less than 1 mm, for example 0.3 mm, when they are placed between the sheet and the mattress. The sensor devices of the invention may be thicker and have different shapes when they are installed within or underneath the mattress in some embodiments of the invention.

Various alternatives and modifications can be made to the aforementioned embodiments. It should further be noted that the embodiments described above may be used in both ways either individually or two or more embodiments may be combined according to user's need. The embodiments should not be limited by their names. For example, the construction of any sensors, e.g. proximity sensors may take on any of a variety of forms, including, but not limited to, an ultrasonic sensor, a capacitive sensor, a photoelectric sensor, an inductive sensor, a camera, a light sensor such as a photodetector, a sound sensor such as a microphone, a mechanical sensor and/or the combination(s) thereof.

The invention has been explained above with reference to the aforementioned embodiments and several commercial and industrial advantages have been demonstrated. The methods and arrangements of the invention allow the measurement of sleep using a sensor device 120 and a mobile third party client application 170 without the user needing to indicate start of sleep manually. This reduces the administrative burden borne by the user in using the system significantly.

The foregoing detailed description will provide those skilled in the art with precise disclosure for implementing an exemplary embodiment of the invention, it being understood that various changes may be made in the methods and order of steps described in an exemplary embodiment without departing from the scope of the invention as set forth in the appended claims. The invention has been explained above with reference to the aforementioned embodiments. However, it is clear that the invention is not only restricted to these embodiments, but includes all possible embodiments within the scope of the inventive thought and the following patent claims.

## Claims

1. A sleep measurement computer system, comprising:
at least one sensor device (120) configured to be installed into a bed (110) of at least one user (220), the at least one sensor device (120) including:
a first sensor (130) configured to:
detect a first type of movement, the first type of movement associated with the at least one user (220) entering the bed (110),
generate a first signal associated with the first type of movement, and
detect a third type of movement, the third type of movement associated with the at least one user (220) exiting the bed (110),
generate a third signal associated with the third type of movement;
wherein the first sensor comprises one of a strain gauge, a capacitive sensor, a proximity sensor, at least one thermocouple, an inductive sensors and a piezo thin film sensor; and
a second sensor (130) configured to:
detect a second type of movement, the second type of movement associated with the at least one user (220) moving while in the bed (110), and
generate a second signal associated with the second type of movement,
wherein the second sensor comprises a piezo thin film sensor, and wherein the first sensor is integrated with the piezo thin film sensor;
a communication subsystem (150) configured to:
initiate a communication channel with a mobile subscriber terminal (180) when the first type of movement is detected, and
transmit the first and second signals from the first and second sensors (130), respectively, to the mobile subscriber terminal (180) through the communication channel; and
the mobile subscriber terminal (180) configured to:
receive the transmitted first and second signals from the at least one sensor device (120) through the communication channel,
start the sleep measurement upon receipt of the first signal from the first sensor (130),
automatically deduce a time of the at least one user (220) in bed (110) based on the first signal,
automatically deduce a starting time of sleep based on the first and second signals,
automatically deduce an end time of sleep,
save data associated with the sleep measurement upon receipt of the second signal, and
automatically ending the sleep measurement upon receipt of the third signal of the first sensor (130).

2. The system of claim 1, wherein the first sensor is the capacitive sensor (310, 320) and includes a capacitive foam material placed between two metal electrode films,
wherein the at least one sensor device (120) is integrated into a fabric of the bed (110).

3. The system of claim 1, wherein the first sensor is the one or more thermocouples (510, 520) and is configured to measure temperature differences between the one or more thermocouples, the measured temperature differences indicating a presence of the at least one user (220).

4. The system of claim 1, wherein the first sensor is the inductive sensor (610, 620) and is configured to detect a presence of the at least one user (220) by creating an AC magnetic field around one or more of the at least one sensor device (120), the at least one user (220), and the bed (110).

5. The system of claim 1, wherein the at least one sensor device (120) includes a sensor matrix placed (710) inside a fabric of the bed (110) and configured to detect a presence of the at least one user (220) on the bed (110).

6. The system of claim 1, wherein the at least one sensor device (120) includes a photodetector configured to detect light intensity levels, the detected light intensity levels associated with the starting time of sleep.

7. The system of claim 1, wherein the at least one sensor device (120) includes a sound detector configured to detect sound intensity levels, the detected sound intensity levels associated with the starting time of sleep.

8. The system of claim 1, wherein the at least one sensor device (120) includes a humidity detector configured to measure a change in air humidity, the detected change in air humidity associated with the starting time of sleep.

9. A sleep recording method to be implemented by a mobile subscriber terminal (180), the method comprising:
detecting a first type of movement, the first type of movement associated with a user (220) entering a bed (110);
in accordance with detecting the first type of movement, generating a first signal associated with the detected first type of movement using a first sensor (130), the first sensor (130) included in at least one sensor device (120)
detecting a third type of movement, the third type of movement associated with the user (220) exiting the bed (110),
in accordance with detecting the third type of movement, generating a third signal associated with the detected third type of movement using the first sensor (130), wherein the first sensor comprises one of a strain gauge, a capacitive sensor, a proximity sensor, at least one thermocouple, an inductive sensors and a piezo thin film sensor;
detecting a second type of movement, the second type of movement associated with the user (220) moving while in bed (110);
in accordance with detecting the second type of movement, generating a second signal associated with the detected second type of movement using a second sensor (130), the second sensor (130) included in the at least one sensor device (120), wherein the second sensor comprises a piezo thin film sensor, and wherein the first sensor is integrated with the piezo thin film sensor;
starting a sleep measurement, the sleep measurement including:
initiating a communication channel between the at least one sensor device (120) and the mobile subscriber terminal (180) when the first type of movement is detected,
communicating the first signal and the second signal from the at least one sensor device (120) to the mobile subscriber terminal (180) through the communication channel,
automatically deducing a time of the user (220) in bed (110) using the first signal,
automatically deducing a start of sleep time using the first and second signals,
automatically deducing an end of the sleep time,
saving data associated with the sleep measurement upon receipt of the second signal from the second sensor (130), and
automatically ending the sleep measurement upon receipt of the third signal of the first sensor (130).

10. The method of claim 9, further comprising:
automatically detecting when the at least one user (220) falls asleep while lying in bed (110).

## Patentansprüche

1. Schlafmessungs-Computersystem, umfassend:
mindestens eine Sensorvorrichtung (120), die konfiguriert ist, um in einem Bett (110) von mindestens einem Benutzer (220) installiert zu werden, die mindestens eine Sensorvorrichtung (120) beinhaltend:
einen ersten Sensor (130), der konfiguriert ist zum:
Erfassen einer ersten Art von Bewegung, wobei die erste Art von Bewegung damit verknüpft ist, dass der mindestens eine Benutzer (220) das Bett (110) betritt,
Erzeugen eines ersten Signals, das mit der ersten Art von Bewegung verknüpft ist, und
Erfassen einer dritten Art von Bewegung, wobei die dritte Art von Bewegung damit verknüpft ist, dass der mindestens eine Benutzer (220) das Bett (110) verlässt,
Erzeugen eines dritten Signals, das mit der dritten Art von Bewegung verknüpft ist;
wobei der erste Sensor eines von einem Dehnungsmessstreifen, einem kapazitiven Sensor, einem Näherungssensor, mindestens einem Thermoelement, einem induktiven Sensor und einem Piezo-Dünnschichtsensor umfasst; und
einen zweiten Sensor (130), der konfiguriert ist zum:
Erfassen einer zweiten Art von Bewegung, wobei die zweite Art von Bewegung damit verknüpft ist, dass sich der mindestens eine Benutzer (220) bewegt, während er sich in dem Bett (110) befindet, und
Erzeugen eines zweiten Signals, das mit der zweiten Art von Bewegung verknüpft ist,
wobei der zweite Sensor einen Piezo-Dünnschichtsensor umfasst, und wobei der erste Sensor mit dem Piezo-Dünnschichtsensor integriert ist;
ein Kommunikationssubsystem (150), das konfiguriert ist zum:
Initiieren eines Kommunikationskanals mit einem mobilen Teilnehmerendgerät (180), wenn die erste Art von Bewegung erfasst wird, und
Übertragen des ersten und zweiten Signals von dem ersten und zweiten Sensor (130) jeweils an das mobile Teilnehmerendgerät (180) über den Kommunikationskanal; und
wobei das mobile Teilnehmerendgerät (180) konfiguriert ist zum:
Empfangen der übertragenen ersten und zweiten Signale von der mindestens einen Sensorvorrichtung (120) über den Kommunikationskanal,
Starten der Schlafmessung bei Empfang des ersten Signals von dem ersten Sensor (130),
automatisches Ableiten einer Zeit des mindestens einen Benutzers (220) im Bett (110) basierend auf dem ersten Signal,
automatisches Ableiten einer Startzeit des Schlafs basierend auf dem ersten und zweiten Signal,
automatisches Ableiten einer Endzeit des Schlafs,
Speichern von Daten, die mit der Schlafmessung verknüpft sind, bei Empfang des zweiten Signals, und
automatisches Beenden der Schlafmessung bei Empfang des dritten Signals des ersten Sensors (130).

2. System nach Anspruch 1, wobei der erste Sensor der kapazitive Sensor (310, 320) ist und ein kapazitives Schaummaterial beinhaltet, das zwischen zwei Metallelektrodenfilmen platziert ist,
wobei die mindestens eine Sensorvorrichtung (120) in ein Gewebe des Bettes (110) integriert ist.

3. System nach Anspruch 1, wobei der erste Sensor das eine oder die mehreren Thermoelemente (510, 520) ist und konfiguriert ist zum Messen von Temperaturunterschieden zwischen dem einen oder den mehreren Thermoelementen, wobei die gemessenen Temperaturunterschiede eine Anwesenheit des mindestens einen Benutzers (220) anzeigen.

4. System nach Anspruch 1, wobei der erste Sensor der induktive Sensor (610, 620) ist und konfiguriert ist, um eine Anwesenheit des mindestens einen Benutzers (220) zu erfassen durch Erzeugen eines magnetischen Wechselfeldes um eine oder mehrere der mindestens einen Sensorvorrichtung (120), des mindestens einen Benutzers (220) und des Bettes (110).

5. System nach Anspruch 1, wobei die mindestens eine Sensorvorrichtung (120) eine Sensormatrix (710) beinhaltet, die innerhalb eines Gewebes des Bettes (110) platziert und konfiguriert ist zum Erfassen einer Anwesenheit des mindestens einen Benutzers (220) auf dem Bett (110).

6. System nach Anspruch 1, wobei die mindestens eine Sensorvorrichtung (120) einen Photodetektor beinhaltet, der konfiguriert ist zum Erfassen von Lichtintensitätspegeln, wobei die erfassten Lichtintensitätspegel mit der Startzeit des Schlafs verknüpft sind.

7. System nach Anspruch 1, wobei die mindestens eine Sensorvorrichtung (120) einen Schalldetektor beinhaltet, der konfiguriert ist zum Erfassen von Schallintensitätspegeln, wobei die erfassten Schallintensitätspegel mit der Startzeit des Schlafs verknüpft sind.

8. System nach Anspruch 1, wobei die mindestens eine Sensorvorrichtung (120) einen Feuchtigkeitsdetektor beinhaltet, der konfiguriert ist zum Messen einer Luftfeuchtigkeitsänderung, wobei die erfasste Luftfeuchtigkeitsänderung mit der Startzeit des Schlafs verknüpft ist.

9. Schlafaufzeichnungsverfahren, das von einem mobilen Teilnehmerendgerät (180) zu implementieren ist, das Verfahren umfassend:
Erfassen einer ersten Art von Bewegung, wobei die erste Art von Bewegung damit verknüpft ist, dass ein Benutzer (220) ein Bett (110) betritt;
gemäß Erfassen der ersten Art von Bewegung, Erzeugen eines ersten Signals, das mit der erfassten ersten Art von Bewegung verknüpft ist, unter Verwenden eines ersten Sensors (130), wobei der erste Sensor (130) in mindestens einer Sensorvorrichtung (120) beinhaltet ist,
Erfassen einer dritten Art von Bewegung, wobei die dritte Art von Bewegung damit verknüpft ist, dass der Benutzer (220) das Bett (110) verlässt,
gemäß Erfassen der dritten Art von Bewegung, Erzeugen eines dritten Signals, das mit der erfassten dritten Art von Bewegung verknüpft ist, unter Verwenden des ersten Sensors (130), wobei der erste Sensor einen Dehnungsmessstreifen, einen kapazitiven Sensor, einen Näherungssensor, mindestens ein Thermoelement, einen induktiven Sensor und einen Piezo-Dünnschichtsensor umfasst;
Erfassen einer zweiten Art von Bewegung, wobei die zweite Art von Bewegung damit verknüpft ist, dass sich der Benutzer (220) bewegt, während er sich im Bett (110) befindet;
gemäß Erfassen der zweiten Art von Bewegung, Erzeugen eines zweiten Signals, das mit der erfassten zweiten Art von Bewegung verknüpft ist unter Verwenden eines zweiten Sensors (130), wobei der zweite Sensor (130) in der mindestens einen Sensorvorrichtung (120) beinhaltet ist, wobei der zweite Sensor einen Piezo-Dünnschichtsensor umfasst, und wobei der erste Sensor mit dem Piezo-Dünnschichtsensor integriert ist;
Starten einer Schlafmessung, die Schlafmessung beinhaltend:
Initiieren eines Kommunikationskanals zwischen der mindestens einen Sensorvorrichtung (120) und dem mobilen Teilnehmerendgerät (180), wenn die erste Art von Bewegung erfasst wird,
Kommunizieren des ersten Signals und des zweiten Signals von der mindestens einen Sensorvorrichtung (120) an das mobile Teilnehmerendgerät (180) über den Kommunikationskanal,
automatisches Ableiten einer Zeit des Benutzers (220) im Bett (110) unter Verwenden des ersten Signals,
automatisches Ableiten eines Starts der Schlafzeit unter Verwenden des ersten und zweiten Signals,
automatisches Ableiten eines Endes der Schlafzeit,
Speichern von Daten, die mit der Schlafmessung verknüpft sind, bei Empfang des zweiten Signals von dem zweiten Sensor (130), und
automatisches Beenden der Schlafmessung bei Empfang des dritten Signals des ersten Sensors (130).

10. Verfahren nach Anspruch 9, ferner umfassend:
automatisches Erfassen wann der mindestens eine Benutzer (220) einschläft, während er im Bett (110) liegt.

## Revendications

1. Un système informatique de mesure du sommeil, comprenant :
au moins un dispositif capteur (120) configuré pour être installé dans un lit (110) d'au moins un utilisateur (220), l'au moins un dispositif capteur (120) comprenant :
un premier capteur (130) configuré pour :
détecter un premier type de mouvement, le premier type de mouvement étant associé à l'au moins un utilisateur (220) qui entre dans le lit (110),
générer un premier signal associé au premier type de mouvement, et
détecter un troisième type de mouvement, le troisième type de mouvement étant associé à l'au moins un utilisateur (220) qui sort du lit (110),
générer un troisième signal associé au troisième type de mouvement,
le premier capteur comprenant l'un d'entre une jauge de contrainte, un capteur capacitif, un capteur de proximité, au moins un thermocouple, un capteur inductif et un capteur à film mince piézoélectrique ; et
un second capteur (130) configuré pour :
détecter un second type de mouvement, le second type de mouvement étant associé à l'au moins un utilisateur (220) qui bouge dans le lit, et
générer un second signal associé au second type de mouvement,
le second capteur comprenant un capteur à film mince piézoélectrique, et le premier capteur étant incorporé au capteur à film mince piézoélectrique ;
un sous-système de communication (150) configuré pour :
établir un canal de communication avec un terminal d'abonné mobile (180) lorsque le premier type de mouvement est détecté, et
transmettre au terminal d'abonné mobile (180) le premier et le second signal provenant, respectivement, du premier et du second capteur (130), par l'intermédiaire du canal de communication ; et
le terminal d'abonné mobile (180), configuré pour :
recevoir le premier et le second signal transmis depuis l'au moins un dispositif capteur (120) par l'intermédiaire du canal de communication,
commencer la mesure du sommeil sur réception du premier signal en provenance du premier capteur,
déduire automatiquement un temps pendant lequel l'au moins un utilisateur (220) était au lit (110), sur la base du premier signal,
déduire automatiquement un instant de début du sommeil, sur la base du premier et du second signal,
déduire automatiquement un temps de fin du sommeil,
sauvegarder des données associées à la mesure du sommeil sur réception du second signal, et
mettre automatiquement fin à la mesure du sommeil sur réception du troisième signal du premier capteur (130).

2. Le système de la revendication 1, dans lequel le premier capteur est le capteur capacitif (310, 320), et il comprend un matériau en mousse capacitif placé entre deux films d'électrode métallique, et dans lequel l'au moins un dispositif capteur (120) est incorporé à un tissu du lit (110).

3. Le système de la revendication 1, dans lequel le premier capteur est ledit un ou plusieurs thermocouples (510, 620), et il est configuré pour mesurer les différentes températures entre les un ou plusieurs thermocouples, les différences de température mesurées indiquant une présence de l'au moins un utilisateur (220).

4. Le système de la revendication 1, dans lequel le premier capteur est le capteur inductif (610, 620) et est configuré pour détecter une présence de l'au moins un utilisateur (220) par création d'un champ magnétique alternatif autour d'un ou plusieurs d'entre l'au moins un dispositif capteur (120), l'au moins un utilisateur (220), et le lit (110).

5. Le système de la revendication 1, dans lequel l'au moins un dispositif capteur (120) comprend une matrice de détecteurs placés (710) à l'intérieur d'un tissu du lit (110) et configurés pour détecter une présence de l'au moins un utilisateur (220) sur le lit (110).

6. Le système de la revendication 1, dans lequel l'au moins un dispositif capteur (120) comprend un photodétecteur configuré pour détecter des niveaux d'intensité lumineuse, les niveaux d'intensité lumineuse étant associés à l'instant de début du sommeil.

7. Le système de la revendication 1, dans lequel l'au moins un dispositif capteur (120) comprend un détecteur de sons configuré pour détecter des niveaux d'intensité sonore, les niveaux d'intensité sonore étant associés à l'instant de début du sommeil.

8. Le système de la revendication 1, dans lequel l'au moins un dispositif capteur (120) comprend un détecteur d'humidité configuré pour mesurer une variation de l'humidité de l'air, le changement détecté de l'humidité de l'air étant associé à l'instant de début du sommeil.

9. Un procédé d'enregistrement du sommeil destiné à être implémenté par un terminal d'abonné mobile (180), le procédé comprenant :
la détection d'un premier type de mouvement, le premier type de mouvement étant associé à un utilisateur (220) qui entre dans un lit (110) ;
suite à la détection du premier type de mouvement, la génération, à l'aide d'un premier capteur (130), d'un premier signal associé au premier type de mouvement détecté, le premier capteur (130) faisant partie d'au moins un dispositif capteur (120) ;
la détection d'un troisième type de mouvement, le troisième type de mouvement étant associé à l'utilisateur (220) qui sort du lit (110) ;
suite à la détection du troisième type de mouvement, la génération, à l'aide du premier capteur (130), d'un troisième signal associé au troisième type détecté de mouvement, le premier capteur comprenant l'un d'entre une jauge de contrainte, un capteur capacitif, un capteur de proximité, au moins un thermocouple, un capteur inductif et un capteur à film mince piézoélectrique ;
la détection d'un second type de mouvement, le second type de mouvement étant associé à l'utilisateur (220) qui bouge dans le lit ;
suite à la détection du second type de mouvement, la génération, à l'aide d'un second capteur (130), d'un second signal associé au second type détecté de mouvement, le second capteur (130) faisant partie de l'au moins un dispositif capteur (120), le second capteur comprenant un capteur à film mince piézoélectrique, et le premier capteur étant intégré au capteur à film mince piézoélectrique ;
le commencement d'une mesure du sommeil, la mesure du sommeil comprenant :
l'établissement d'un canal de communication entre l'au moins un dispositif capteur (120) et le terminal d'abonné mobile (180) lorsque le premier type de mouvement est détecté,
la communication du premier signal et du second signal de l'au moins un dispositif capteur (120) au dispositif d'abonné mobile (180) par l'intermédiaire du canal de communication,
la déduction automatique, à l'aide du premier signal, d'un temps passé au lit (110) par l'utilisateur (220),
la déduction automatique d'un instant de début de sommeil,
la déduction automatique, à l'aide du premier et du second signal, d'un instant de fin du sommeil,
la sauvegarde de données associées à la mesure du sommeil sur réception du second signal en provenance du second capteur (130), et
l'achèvement automatique de la mesure du sommeil sur réception du troisième signal du premier capteur (130).

10. Le procédé de la revendication 9, comprenant en outre :
la détection automatique du moment où l'au moins un utilisateur (220) s'endort couché dans son lit (110).
